Europäisches Patentamt

European Patent Office

Office européen des brevets

(19)

(11) Veröffentlichungsnummer: **0 273 314**
**A2**

## (12) EUROPÄISCHE PATENTANMELDUNG

(21) Anmeldenummer: 87118795.1

(22) Anmeldetag: 18.12.87

(51) Int. Cl.4: **C07C 67/29** , C07C 69/14 , C07C 69/24

(30) Priorität: 23.12.86 DE 3644837

(43) Veröffentlichungstag der Anmeldung:
06.07.88 Patentblatt 88/27

(84) Benannte Vertragsstaaten:
CH DE FR GB LI NL

(71) Anmelder: HOECHST AKTIENGESELLSCHAFT
Postfach 80 03 20
D-6230 Frankfurt am Main 80(DE)

(72) Erfinder: Driscoll, Robert Kenneth, Dr.
Heilmann-Strasse 43
D-6000 Frankfurt am Main 50(DE)
Erfinder: Leupold, Ernst Ingo, Dr.
Am Zäunefeld 15
D-6392 Neu-Anspach(DE)

(54) Verfahren zur Herstellung von Acyloxyacetaldehyden.

(57) Die Erfindung betrifft ein Verfahren zur Herstellung von Acyloxyacetaldehyden R-COOCH$_2$-CHO wobei R ein geradkettiger oder verzweigter Alkylrest mit 1 bis 8 C-Atomen ist, durch katalytische Oxidehydrierung von Acyloxyethanolen in der Gasphase. Die Katalysatoren enthalten Silber und SiO$_2$, Al$_2$O$_3$ oder Aluminiumsilicat.

FIG. 1

EP 0 273 314 A2

## Verfahren zur Herstellung von Acyloxyacetaldehyden

Die Erfindung betrifft ein Verfahren zur Herstellung von Acyloxyacetaldehyden durch katalytische Oxidehydrierung von Acyloxyethanolen in der Gasphase gemäß der allgemeinen Gleichung:

$$R\text{-}COOCH_2CH_2OH + 1/2\ O_2 \xrightarrow{\text{Kat.}} R\text{-}COOCH_2CHO + H_2O$$

Aus US-PS 2 286 034 ist bereits bekannt, daß Acyloxyacetaldehyde durch katalytische Dehydrierung der entsprechenden Acyloxyethanole hergestellt werden können. Dabei wird der Alkohol in Abwesenheit von Sauerstoff an einem reduzierten $CuO/SiO_2/CrO_3$-Katalysator dehydriert. Dieser Katalysator enthält ein saures Oxid, nämlich $SiO_2$, und liefert nur geringe Ausbeuten an verschiedenen Aldehyden.

Aus DE-OS 2 049 162 ist dieselbe katalytische Dehydrierung bei relativ niedrigen Temperaturen (150 bis 350°C) bekannt. Die besten hier beschriebenen Katalysatoren enthalten Ag, Cu oder Messing sowie basische Metalloxide, insbesondere MgO. Vom Zusatz saurer Oxide, wie Siliciumdioxid oder Aluminiumoxid wird dagegen abgeraten, wegen möglicher unerwünschter Spalt-und Nebenreaktionen (Seite 2, Zeilen 5 bis 8). Die Dehydrierung wird in Anwesenheit von gasförmigen basischen Stickstoffverbindungen, wie Ammoniak oder Aminen durchgeführt. Sauerstoff kann dabei anwesend sein. Bei der Herstellung von Acetoxyacetaldehyd wird eine Ausbeute von 56 % bei 72 %igem Umsatz beschrieben.

Es wurde nun gefunden, daß hohe Ausbeuten an Acyloxyacetaldehyden erreicht werden durch Oxidehydrierung der entsprechenden Acyloxyethanole an Katalysatoren, die Silber und bestimmte saure Komponenten enthalten, nämlich $SiO_2$, $Al_2O_3$ oder Aluminiumsilicat oder deren Gemische, im folgenden auch kurz als "saure Komponenten" bezeichnet.

In der obengenannten DE-OS 2 049 162 wurden basische Oxide und basische Stickstoffverbindungen verwendet, um saure Zentren am Katalysator zu vermeiden bzw. zu neutralisieren. Der saures Oxid enthaltende Katalysator gemäß der obengenannten US-PS 2 286 034 ergab schlechte Ausbeuten. Es ist daher sehr überraschend, daß gute Ausbeuten mit einem saures Oxid oder (saures) Aluminiumsilicat enthaltenden Katalysator erhalten werden.

Gegenstand der vorliegenden Erfindung ist daher ein Verfahren zur Herstellung von Acyloxyacetaldehyden der Formel $RCOOCH_2CHO$, wobei R ein geradkettiger oder verzweigter Alkylrest mit 1 bis 8 C-Atomen ist, durch Oxidehydrierung der entsprechenden Acyloxyethanole in der Gasphase an einem silberhaltigen Katalysator, dadurch gekennzeichnet, daß der Katalysator zusätzlich $SiO_2$, $Al_2O_3$ oder Aluminiumsilicat oder deren Gemische enthält. Vorzugsweise arbeitet man dabei in Abwesenheit von gasförmigen basischen Stickstoffverbindungen.

Bei dem erfindungsgemäßen Verfahren werden Acyloxyethanole der allgemeinen Formel $R\text{-}COOCH_2CH_2OH$ in Dampfform eingesetzt. Dabei ist R ein geradkettiger oder verzweigter Alkylrest mit 1 bis 8 C-Atomen, vorzugsweise 1 bis 4 C-Atomen.

Die Acyloxyethanole sind z.B. durch Umsetzung von Ethylenoxid mit den entsprechenden Carbonsäuren in Anwesenheit eines geeigneten Katalysators in hohen Ausbeuten zugänglich (DRP 515306). Gemische aus Acyloxyethanolen und Diacyloxyethanen, die z.B. bei der Veresterung von Säuren mit Ethylenglykol entstehen, können ebenfalls als Edukt verwendet werden. Der Einsatz von reinen Acyloxyethanolen wird jedoch bevorzugt.

Erfindungsgemäß werden die Acyloxyethanole in Dampfform zusammen mit Sauerstoff über den Katalysator geleitet. In vielen Fällen hat es sich als zweckmäßig erwiesen, unter Zumischung eines Inertgases zu arbeiten. Als Inertgas kommen z.B. Stickstoff, Edelgase und Kohlendioxid infrage. Falls man Luft verwendet, erfüllt diese gleichzeitig die Funktion des Inertgases und der $O_2$-Quelle. In den meisten Fällen ist die gleichzeitige Zufuhr von Wasserdampf in den Reaktionsraum vorteilhaft.

Bei dem erfindungsgemäßen Verfahren setzt man pro Mol Acyloxyethanol folgende Mengen an Zusatzstoffen ein:

Inertgas: 0 bis 80 Mol
Sauerstoff: 0,1 bis 20 Mol, vorzugsweise 0,5 bis 2,5 Mol
Wasser: 0 bis 10 Mol, vorzugsweise 0 bis 3 Mol

Die saure Komponente wird vorzugsweise gleichzeitig als Träger benutzt. Vorzugsweise verwendet man in diesem Fall Siliciumdioxid, Aluminiumoxid oder Aluminiumsilicat mit einer BET-Oberfläche unterhalb 100 $m^2g^{-1}$, insbesondere unterhalb 30 $m^2g^{-1}$. Besonders bevorzugt als Träger ist Aluminiumsilicat mit einer BET-Oberfläche unterhalb 30 $m^2g^{-1}$.

Vorzugsweise enthält der Katalysator zusätzlich noch Phosphor. Die Menge an Silber bzw. an Phosphor kann in weiten Grenzen schwanken. Im allgemeinen beträgt sie 0,01 bis 50 Gew.-%, vorzugsweise 0,1 bis 20 Gew.-%, bezogen auf die Gesamtmasse des Katalysators. Dabei beträgt das Atomverhältnis Ag : P im allgemeinen 1 : 0,01 bis 1 : 10, vorzugsweise 1 : 0,1 bis 1 : 10.

Ag wird entweder in metallischer Form oder in Form einer Verbindung, z.B. als Nitrat, Phosphat, Chlorat, Acetat, Lactat, Propionat oder Tartrat in den Katalysator eingebracht. P wird entweder als Element oder vorzugsweise in Form einer Verbindung, z.B. als Phosphat oder als eine der Phosphorsäuren eingesetzt.

Silber und Phosphor werden zweckmäßig in Form einer Lösung auf die als Träger benutzte saure Komponente aufgebracht; dann wird das Lösemittel abgedampft und der Katalysator getrocknet. Als Lösemittel verwendet man im allgemeinen Wasser, Salzsäure, Salpetersäure, Alkalilaugen oder Ammoniaklösung.

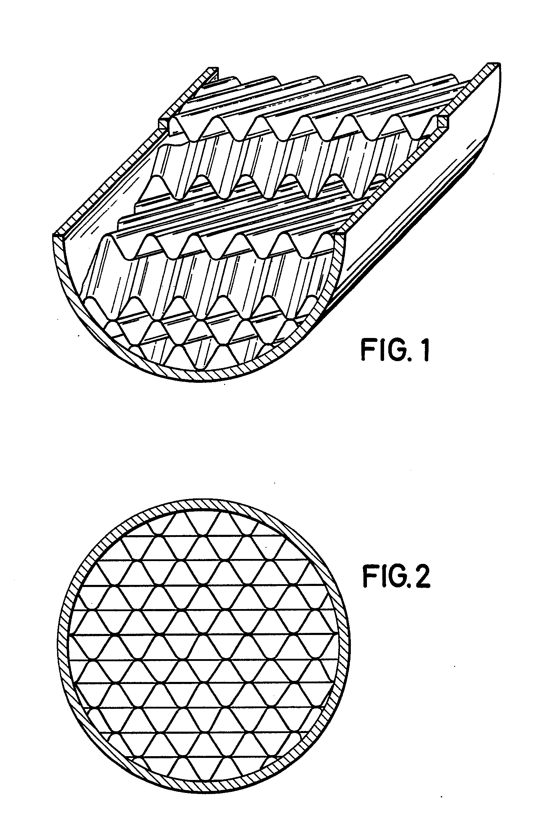

Das Trägermaterial kann in vielen verschiedenen geometrischen Formen eingesetzt werden. Besonders geeignete Formen sind z.B. Kugeln oder die in der DE-OS 3 401 115 beschriebenen Monolithstrukturen, die schon bei statischen Mischern bekannt sind. Diese Monolithstrukturen sind in den Abbildungen 1 und 2 wiedergegeben. Solche Monolithe, die im folgenden auch als Monolithe Typ I bezeichnet werden, bestehen aus parallel zur Reaktorachse angeordneten, aufeinanderliegenden gewellten Schichten (also etwa wie Wellblech geformt), wobei die Wellentäler in aufeinanderfolgenden Schichten alternierend einen Winkel von $\alpha$ und $-\alpha$ mit der Reaktorachse bilden ($\alpha = 20 - 70°$). Die durch die Wellentäler und Wellenkämme der aufeinanderliegenden Schichten gebildeten Kanäle haben den Querschnitt eines Dreiecks mit stark abgerundeten Ecken. Zwischen den aufeinanderliegenden gewellten Schichten existiert an jeder Berührungsstelle eine feste Verbindung, so daß jedes Monolithelement eine einzige mit Kanälen durchzogene Hohlraumstruktur darstellt. Im allgemeinen werden die Monolithe in Form von mehreren aufeinander gestapelten Zylindern eingesetzt. Die beschriebene Struktur stellt dabei automatisch sicher, daß die Kanäle der aufeinandergestapelten Zylinder so zueinander passen, daß das eingesetzte Gas vom Eingang des Reaktorrohrs zum Ausgang fließen kann.

Solche Monolithkatalysatoren eignen sich z.B. für den Einsatz in größeren Reaktorröhren, u.a. weil der Druckverlust in der Katalysatorschüttung bei kürzeren Verweilzeiten sehr niedrig ist und weil die Reaktionswärme effektiv auf die gekühlte Reaktorwand übertragen wird.

Es hat sich als vorteilhaft erwiesen, vor dem Einleiten des Acetoxyethanols in die Reaktionszone ein oxidierendes Gas, insbesondere Sauerstoff oder Luft, bei Temperaturen von 100 bis 800°C, insbesondere 200 bis 600°C, über den Katalysator zu leiten.

Das erfindungsgemäße Verfahren wird im allgemeinen bei Temperaturen zwischen 300 und 700°C, vorzugsweise zwischen 360 und 550°C durchgeführt. Die Verweilzeit liegt vorzugsweise zwischen 0,01 und 10 Sekunden, insbesondere jedoch zwischen 0,05 und 1 Sekunde. Im allgemeinen werden mit zunehmender Reaktionstemperatur kürzere Verweilzeiten gewählt.

Das erfindungsgemäße Verfahren wird bevorzugt bei Normaldruck durchgeführt, jedoch können auch verminderte oder erhöhte Drucke verwendet werden (0,01 bis 100 bar).

Im einzelnen geht man so vor, daß das Acyloxyethanol bzw. das Gemisch aus Acyloxyethanol und Wasser aus einer Dosiervorrichtung in eine Verdampfungszone und das entstandene Gas dann durch ein von außen beheiztes und mit dem Katalysator gefülltes Reaktionsrohr geleitet wird. In der Verdampfungszone erfolgt gegebenenfalls die Vermischung mit dem Inertgas und/oder dem Sauerstoff oder dem sauerstoffhaltigen Gas; es hat sich als vorteilhaft erwiesen, diese Gase vor der Vermischung auf die Reaktionstemperatur vorzuheizen. Die Reaktionsprodukte werden nach Verlassen des Reaktors zur Abtrennung der kondensierbaren Anteile gekühlt.

Die Verfahrensprodukte sind wertvolle Zwischenprodukte, z.B. für die Herstellung von thermoplastischen Harzen oder heterocyclischen Verbindungen. Acetoxyacetaldehyd wird bei der Herstellung von Tulipalin B verwendet (J. Med. Chem. 29, S. 868 - 871, 1986). Diese Substanz ist als Bakterizid, Fungicid und Hautsensibilisator bekannt.

Die nachfolgenden Beispiele sollen die Erfindung näher erläutern.

**Beispiel 1**

Zur Herstellung des Katalysators wurden 50 g eines kugelförmigen Aluminiumsilicat-Trägers (BET-Oberfläche 1 $mg^2/g$) mit einer Lösung von 2,5 g $Ag_3PO_4$ in 17,5 ml konzentriertem Ammoniak getränkt und das Lösemittel auf dem Dampfbad abgedampft. Der Katalysator wurde bei 110°C getrocknet. Anschließend wurde in gleicher Weise eine Lösung von 1,19 g kristalliner $H_3PO_4$ in 13,8 ml Wasser aufgebracht und der Katalysator erneut bei 110°C getrocknet. Der Katalysator enthielt 3,87 Gew.-% Ag und 1,13 Gew.-% P, was einem Ag/P-Atomverhältnis von 1 : 1 entspricht.

10 ml dieses Katalysators wurden in ein von außen beheiztes Glasreaktorrohr gefüllt und wie folgt kalziniert: Der Katalysator wurde innerhalb von 6 Std. langsam in einem Gasstrom aus 7,3 Nl/h Sauerstoff und 56 Nl/h Stickstoff auf 450°C erhitzt.

Anschließend wurden 4,9 g/h eines Gemisches aus Glykolacetat (91,9 Gew.-%) und Wasser (8,1 Gew.-%) mit Hilfe einer Kolbenspritze über eine Verdampfungszone in ein senkrecht angeordnetes Reaktionsrohr (Länge: 15 cm, Durchmesser: 2 cm) eingeleitet. Gleichzeitig wurden 60 Nl/h Stickstoff und 1,45 Nl/h Sauerstoff zugeführt, die beide vorher auf 435°C aufgeheizt worden waren. Die Reaktionstemperatur von 435°C wurde mit Hilfe eines beweglichen Thermoelementes im Inneren des Reaktors gemessen. Die Reaktionsprodukte wurden in einer Kühlfalle bei -70°C kondensiert und mit gaschromatographischen Methoden analysiert.

Nach einer Anlaufzeit von 1 Stunde zur Einstellung konstanter Betriebsbedingungen wurde der eigentliche Versuch über einen Zeitraum von 2 Stunden durchgeführt. Als Nebenprodukte wurden Essigsäure, Formaldehyd, Acetaldehyd, CO und $CO_2$ gefunden.

Als Ergebnis des zweistündigen Versuches wurden 69,4 mmol Acetoxyacetaldehyd und 8,9 mmol Glykolacetat im Kondensat gefunden, was einer 80,1 %igen Ausbeute bei 89,7 %igem Umsatz entspricht.

**Beispiel 2**

Analog zu Beispiel 1 wurden in die dort beschriebene Apparatur 5,7 g/h Glykolisobutyrat ($CH_3CH(CH_3)$-$COOCH_2CH_2OH$), 0,4 g/h Wasser, 90 Nl/h Stickstoff und 1,2 Nl/h Sauerstoff eingeleitet. Der Katalysator enthielt 3,87 Gew.-% Ag und 0,57 Gew.-% P auf demselben Aluminiumsilicat-Träger wie im Beispiel 1 und war in gleicher Weise hergestellt und kalziniert worden. Es wurden 10 ml dieses Katalysators eingesetzt. Die Reaktionstemperatur betrug 485°C.

Als Ergebnis eines zweistündigen Versuches wurden 66,9 mmol iso-Butyroxyacetaldehyd und 4,8 mmol Glykolisobutyrat im Kondensat gefunden, was einer 77,2 %igen Ausbeute bei 94,5 %igem Umstz entspricht.

**Beispiel 3**

Zur Herstellung des Katalysators wurden 50 g desselben Aluminiumsilicat-Trägers wie im Beispiel 1 mit einer Lösung von 3,06 g $AgNO_3$ in 13,5 ml Wasser getränkt und das Lösemittel auf dem Dampfbad abgedampft. Anschließend wurde der Katalysator bei 110°C getrocknet und analog zu Beispiel 1 kalziniert.

Analog zu Beispiel 1 wurden in die dort beschriebene Apparatur 4,5 g/h Glykolacetat, 0,2 g/h $H_2O$, 60 Nl/h Stickstoff und 1,45 Nl/h Sauerstoff eingeleitet. 10 ml dieses Katalysators wurden eingesetzt. Die Reaktionstemperatur betrug 450°C.

Als Ergebnis eines zweistündigen Versuches wurden 59,2 mmol Acetoxyacetaldehyd und 12,6 mmol Glykolacetat im Kondensat gefunden, was einer 68,4 %igen Ausbeute bei 85,4 %igem Umsatz entspricht.

**Beispiel 4**

Der Versuch wurde in einem senkrecht angeordneten V4A-Stahlrohrreaktor von 150 cm Länge und 2,7 cm Durchmesser durchgeführt. Der Reaktor wurde von außen beheizt und die Temperatur im Inneren mit Hilfe mehrerer Thermoelemente gemessen.

Der Katalysatorträger bestand aus 30 aufeinandergeschichteten zylinderförmigen Monolithstücken des Typs I. Jedes Stück hatte einen Durchmesser von 26 mm, eine Länge von 50 mm und ca. 3 mm Kanaldurchmesser. Zentral durch die Monolith-Körper war eine Bohrung von 9 mm Durchmesser durchgeführt, in welche Thermoelemente zur Temperaturmessung eingepaßt waren. Die Monolithe bestanden aus mit Aluminiumoxid beschichtetem Aluminiumsilikat mit ca. 20 $m^2g^{-1}$ BET-Oberfläche. Der Katalysatorträger füllte ein Volumen von 700 ml im Reaktorrohr aus. Dieses Volumen wird im folgenden als Katalysatorvolumen bezeichnet.

Zur Herstellung des Katalysators wurde jedes Monolithstück durch Eintauchen in eine heiße 4,5 molare Lösung von Silbernitrat in Wasser getränkt. Das Lösungsmittel wurde auf einem Dampfbad abgedampft und der Monolith anschließend bei 110°C getrocknet. Dieses Verfahren wurde wiederholt, bis die gewünschte Silber-Konzentration, nämlich 25,0 g Silber pro Liter Katalysatorvolumen erreicht war.

In gleicher Weise wurden 7,1 g Phosphor pro Liter Katalysatorvolumen auf den bereits mit Silber getränkten Monolith durch Eintauchen in eine 4,8 molare wäßrige Lösung von ortho-Phosphorsäure aufgebracht.

Der gesamte Katalysator wurde innerhalb von 6 Std. langsam im einem Gasstrom aus 22 mol/h Sauerstoff und 175 mol/h Stickstoff auf 450°C erhitzt.

3,4 mol/h Glykolacetat, 1,7 mol/h Wasser, 5,0 mol/h Sauerstoff und 187,5 mol/h Stickstoff wurden gleichzeitig in einen Verdampfungstopf eingeleitet. Die gesamte Gasphase wurde vor Einleitung in den senkrecht angeordneten Reaktor auf 435°C aufgeheizt. Die Reaktionstemperatur im Reaktorrohr betrug ebenfalls 435°C. Nach einer Anlaufzeit von 1 Stunde zur Einstellung konstanter Betriebsbedingungen wurde der Versuch über einen Zeitraum von 4 Stunden durchgeführt.

Die folgenden Ergebnisse wurden erhalten:

Acetoxyacetaldehyd-Ausbeute = 81,9 %

Glykolacetat-Umsatz = 90,3 %.

## Ansprüche

1. Verfahren zur Herstellung von Acyloxyacetaldehyden der Formel $R-COOCH_2CHO$, wobei R ein geradkettiger oder verzweigter Alkylrest mit 1 bis 8 C-Atomen ist, durch Oxidehydrierung der entsprechenden Acyloxyethanole in der Gasphase an einem silberhaltigen Katalysator, dadurch gekennzeichnet, daß der Katalysator zusätzlich Siliciumdioxid, Aluminiumoxid oder Aluminiumsilicat oder deren Gemische enthält.

2. Verfahren nach Anspruch 1, dadurch gekennzeichnet, daß das Aluminiumoxid, Siliciumdioxid oder Aluminiumsilicat als Träger für das Silber benutzt wird und eine BET-Oberfläche von weniger als 100 $m^2g^{-1}$ hat.

3. Verfahren nach Anspruch 1 oder 2, dadurch gekennzeichnet, daß der Katalysator zusätzlich Phosphor enthält.

4. Verfahren nach mindestens einem der Ansprüche 1 bis 3, dadurch gekennzeichnet, daß die Reaktionstemperatur zwischen 300 und 700°C liegt.

5. Verfahren nach mindestens einem der Ansprüche 1 bis 4, dadurch gekennzeichnet, daß die Verweilzeit zwischen 0,01 und 10,0 Sekunden liegt.

FIG. 1

FIG. 2